(19) Europäisches Patentamt
European Patent Office
Office européen des brevets

(11) Publication number: **0 012 729**
**B1**

(12) # EUROPEAN PATENT SPECIFICATION

(45) Date of publication of patent specification: **14.07.82**

(51) Int. Cl.³: **C 07 C 57/50, A 61 K 31/19**

(21) Application number: **79830049.7**

(22) Date of filing: **05.12.79**

(54) **Therapeutic compositions containing as active compound a substituted acetic acid or its salt.**

(30) Priority: **06.12.78 IT 3063078**

(43) Date of publication of application:
**25.06.80 Bulletin 80/13**

(45) Publication of the grant of the patent:
**14.07.82 Bulletin 82/28**

(84) Designated Contracting States:
**AT BE CH DE FR GB NL SE**

(56) References cited:
**FR - M - 8 494**

**TETRAHEDRON LETTERS, no. 28, june 1969, pages 2331—2334 S.K. DASGUPTA et al.: "A novel synthesis of styrylacetic acids systems"**

(73) Proprietor: **"IBIS" ISTITUTO BIOCHIMICO SPERIMENTALE S.p.A.**
**Viale Macchiavelli, 31**
**I-50125 Firenze (IT)**

(72) Inventor: **Chiti, Walter**
**Via Benedetto da Foiano, 11**
**Firenze (IT)**
Inventor: **Allodi, Germano**
**Via Gagliano, 49**
**Firenze (IT)**

(74) Representative: **Notarbartolo, Manfredi, et al,**
**Viale Bianca Maria, 33**
**Milano (IT)**

**EP 0 012 729 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Therapeutic compositions containing as active compound a substituted acetic acid or its salt

This invention relates to the use in human therapy of α-methyl-α-(3,4-dihydro-1-naphthyl)-acetic acid of formula

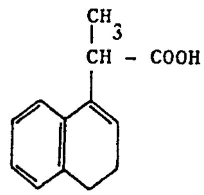

(I)

α-methyl-α-(3,4-dihydro-1-naphthyl)-acetic acid has already been prepared (J. Chem. Soc. 2799—2805 (1969) during the course of a purely chemical study of β-hydroxy acids.

No possibility of direct industrial or human therapy use for this compound has ever been indicated.

It has now been quite unexpectedly found that compound (I) has strong choleretic activity accompanied by very low acute toxicity and by the absence of chronic toxicity during prolonged administration at therapeutic doses, and this forms the subject matter of the present invention. In comparison with the best known choleretics, compound (I) represents considerable and unforeseeable progress from many aspects.

It is known that the primary effect of a choleretic agent is to increase the bile flow and the quantity of solid substances excreted with the bile. A good choleretic must provide this effect in a sufficiently intense and prolonged manner. In addition, as in the case of all drugs, it must have low acute toxicity and good general tolerability, but in particular it must be free from toxic effects on the organ on which it is required to act, i.e. the liver, whether this is in a normal or pathological state. The analysis of the new product in comparison with the best known products for the same therapeutic use has therefore been focused on these aspects. The choleretic activity of the new product has been determined in the rat by using the bile fistula method. Wistar rats of both sexes and having a weight of between 180 and 240 g were used.

The animals, which were kept fasting for 12 hours but with free access to water, were narcotised by ethyl urethane in a dose of 1 g/kg by intraperitoneal or intramuscular injection.

After binding the animals to the retention bed, a median laparatomy was carried out, and when the bile duct had been isolated a cannula needle was inserted into it. A polyethene tube was then inserted into those animals which were to receive the products intraduodenally, after which a suture was carried out on the abdominal wall, and the bile was allowed to discharge for 30 minutes after inserting the cannula in the bile duct, so as to allow the discharge to become normal. The bile was then collected for one hour.

Having determined the volume of bile discharged during this first hour of collection (base volume), the products under examination were administered at the predetermined doses and by the predetermined methods. The bile excreted by the individual animals was then collected over each hour for a further six hours.

During the experiments, the animals were kept at ambient temperature of about 22°C, and at regular intervals of two hours received 10 ml/kg of physiological solution s.c.

The liquids were administered at a temperature of 37°C.

The bile flow and cholesterol excretion were determined on those bile fractions excreted by the animals over each hour starting from the base hour until the end of the experiment.

The dry residue (R.S.) was determined on the bile fraction excreted during the base hour and on the total bile fractions excreted during the subsequent hours. For practical reasons, the determination of the dry residue and of the cholesterol (carried out by the Sperry & Webb method) were carried out on the pool of bile excreted by the entire group of animals subjected to the same treatment instead of on the bile fractions excreted by the individual animals (which was sometimes insufficient for determining the cholesterol).

The choleretic according to the invention was administered in its pure state, as the sodium salt of α-methyl-α-(3,4-dihydro-1-naphthyl)-acetic acid (symbol 1.620) in aqueous solution, and as a formulation of the following composition

| | |
|---|---|
| 1.620 | 100 mg |
| lactose | 80 mg |
| sodium stearate | 20 mg |

Said formulation, indicated for the brevity by the symbol CO was administered in 10% gum arabic mucilage.

The following were used as comparison products:
dehydrocholic acid in the form of an aqueous solution of its sodium salt; α-(1-hydroxy-4-phenylcyclohexyl)butyric acid also in the form of an aqueous solution of its sodium salt; and dihydroxybutylether in the form of an aqueous emulsion.

For the controls, a physiological solution or gum arabic mucilage was used in the same volumes as used for administering the drugs. In a first series of experiments, an evaluation was made of the effect of the compound 1.620 administered orally at progressive doses on choleresis, on the bile flow and on the dry residue (R.S.).

The results obtained are shown in Table 1.

The values given are mean values (± s.e.) for groups of ten animals.

For comparison purposes, the same determinations were repeated with dehydrochloric acid (also administered orally in progressive doses) and with two different choleretics of current therapeutic use, namely α-(1-hydroxy-4-phenylcyclohexyl)butyric acid and dihydroxybutylether.

The results of this series of experiments are given in Table 2, in terms of the mean value (± s.e.) of tests carried out on groups of ten animals.

In the tables, the bile flow is expressed in ml/kg/h and the dry residue in mg/ml and in mg/kg/h.

The action of the compound 1.620 on choleresis (flow and R.S.) was also determined by intraduodenal administration of increasing doses, in comparison with dehydrocholic acid administered fin the same way. Mean value (± s.e.) for groups of 10 animals are given in table 3.

TABLE 1

| Bile excretion at the times indicated | | 0 | 1h | 2h | 3h | 4h | 5h | 6h |
|---|---|---|---|---|---|---|---|---|
| Controls | Flow (ml/kg/h) | 2,77 ± 0,14 | 2,81 ± 0,18 | 2,76 ± 0,13 | 2,56 ± 0,15 | 2,39 ± 0,15 | 2,00 ± 0,13 | 1,95 ± 0,13 |
| 5 cc/kg S.F. | | | | | | | | |
| p.o. | R.S. mg/ml | 31,8 | 29,0 | | | 25,6 | | |
| | R.S. mg/kg/h | 88,2 | 81,5 | | | 59,7 | | |
| 1.620 | Flow (ml/kg/h) | 3,05 ± 0,33 | 3.89 + 0,47 | 3,13 ± 0,24 | 2,84 ± 0,21 | 2,35 ± 0,19 | 2,05 ± 0,21 | 1,87 ± 0,39 |
| 25 mg/kg | | | | | | | | |
| p.o. | R.S. mg/ml | 27,8 | 25,5 | | | 23,2 | | |
| | R.S. mg/kg/h | 85,0 | 99,2 | | | 56,7 | | |
| 1.620 | Flow (ml/kg/h) | 2,76 ± 0,09 | 4,90 ± 0,16 | 3,74 ± 0,13 | 3,34 ± 0,13 | 2,73 ± 0,07 | 2,60 ± 0,08 | 2,25 ± 0,09 |
| 50 mgKg | | | | | | | | |
| p.o. | R.S. mg/ml | 29,4 | 25,2 | | | 23,2 | | |
| | R.S. mg/kg/h | 81,3 | 123,7 | | | 67,9 | | |
| 1.620 | Flow (ml/kg/h) | 2,40 ± 0,23 | 4,44 ± 0,28 | 4,95 ± 0,39 | 4,10 ± 0,32 | 3,10 ± 0,31 | 2,79 ± 0,23 | 2,33 ± 0,25 |
| 100 mg/kg | | | | | | | | |
| p.o. | R.S. mg/ml | 28,1 | 24,0 | | | 19,8 | | |
| | R.S. mg/kg/h | 67,6 | 107,0 | | | 68,6 | | |
| 1.620 | Flow (ml/kg/h) | 2,46 ± 0,12 | 5,53 ± 0,30 | 5,78 ± 0,29 | 5,02 ± 0,34 | 4,77 ± 0,57 | 4,19 ± 0,25 | 3,39 ± 0,14 |
| 200 mg/kg | | | | | | | | |
| p.o. | R.S. mg/ml | 28,4 | 24,7 | | | 21,8 | | |
| | R.S. mg/kg/h | 70,0 | 137,0 | | | 101,0 | | |

TABLE 2

| Bile excretion at the times indicated | | 0 | 1h | 2h | 3h | 4h | 5h | 6h |
|---|---|---|---|---|---|---|---|---|
| Dehydrocholic acid | Flow (ml/kg/h) | 2,46 ± 0,05 | 3,33 ± 0,14 | 1,88 ± 0,14 | 2,19 ± 0,15 | 1,92 ± 0,17 | 1,77 ± 0,16 | 1,29 ± 0,15 |
| 25 mg/kg | R.S. mg/ml | 24,3 | 25,2 | | | 22,6 | | |
| p.o. | R.S. mg/kg/h | 59,8 | 84,1 | | | 41,0 | | |
| Dehydrocholic acid | Flow (ml/kg/h) | 2,23 ± 0,22 | 3,75 ± 0,75 | 2,60 ± 0,24 | 2,15 ± 0,28 | 2,10 ± 0,24 | 1,64 ± 0,20 | 1,64 ± 0,24 |
| 100 mg/kg | R.S. mg/ml | 25,0 | 26,4 | | | 22,5 | | |
| p.o. | R.S. mg/kg/h | 55,4 | 98,6 | | | 45,5 | | |
| Dehydrocholic acid | Flow (ml/kg/h) | 2,75 ± 0,17 | 5,39 ± 0,24 | 3,50 ± 0,13 | 2,64 ± 0,19 | 2,78 ± 0,18 | 2,61 ± 0,13 | 2,38 ± 0,14 |
| 200 mg/kg | R.S. mg/ml | 31,2 | 31,9 | | | 26,9 | | |
| p.o. | R.S. mg/kg/h | 85,8 | 171,9 | | | 74,9 | | |
| Dehydrocholic acid | Flow (ml/kg/h) | 2,53 ± 0,10 | 7,68 ± 0,20 | 4,70 ± 0,45 | 3,06 ± 0,15 | 2,59 ± 0,16 | 2,29 ± 0,12 | 1,97 ± 0,15 |
| 400 mg/kg | R.S. mg/ml | 28,3 | 31,0 | | | 24,9 | | |
| p.o. | R.S. mg/kg/h | 71,8 | 238,6 | | | 77,8 | | |
| α-(1-hydroxy-4-phenyl-cyclohexyl)butyric acid | Flow (ml/kg/h) | 2,70 ± 0,25 | 5,60 ± 0,51 | 5,42 ± 0,47 | 4,63 ± 0,28 | 3,68 ± 0,18 | 3,05 ± 0,12 | 2,70 ± 0,17 |
| 200 mg/kg | R.S. mg/ml | 30,8 | 24,6 | | | 23,0 | | |
| p.o. | R.S. mg/kg/h | 84,1 | 138,9 | | | 90,8 | | |
| Dihydroxydibutylether | Flow (ml/kg/h) | 3,36 ± 0,19 | 5,10 ± 0,23 | 3,79 ± 0,18 | 3,08 ± 0,15 | 2,68 ± 0,15 | 1,97 ± 0,05 | 1,77 ± 0,11 |
| 200 mg/kg | R.S. mg/ml | 28,7 | 26,7 | | | 24,0 | | |
| p.o. | R.S. mg/kg/h | 96,2 | 135,6 | | | 63,5 | | |

TABLE 3

| Treatment | | Bile excretion at the times indicated 0 | 1h | 2h | 3h | 4h | 5h | 6h |
|---|---|---|---|---|---|---|---|---|
| Controls | Flow (ml/kg/h) | 2,46 ± 0,17 | 2,63 ± 0,16 | 2,41 ± 0,15 | 2,26 ± 0,16 | 2,28 ± 0,17 | 2,15 ± 0,19 | 1,97 ± 0,19 |
| 2,5 cc/kg S.F. | R.S. mg/ml | 27,0 | 23,1 | | | 22,1 | | |
| i.d. | R.S. mg/kg/h | 66,5 | 60,9 | | | 46,8 | | |
| 1.620 | Flow (ml/kg/h) | 2,66 ± 0,16 | 4,24 ± 0,25 | 3,21 ± 0,10 | 2,89 ± 0,10 | 2,44 ± 0,15 | 2,20 ± 0,14 | 1,68 ± 0,05 |
| 25 mg/kg | R.S. mg/ml | 31,4 | 27,9 | | | 24,4 | | |
| i.d. | R.S. mg/kg/h | 83,3 | 119,7 | | | 60,0 | | |
| 1.620 | Flow (ml/kg/h) | 2,04 ± 0,11 | 3,90 ± 0,28 | 3,57 ± 0,16 | 2,69 ± 0,09 | 2,20 ± 0,07 | 2,00 ± 0,10 | 1,63 ± 0,08 |
| 50 mg/kg | R.S. mg/ml | 29,4 | 24,3 | | | 23,0 | | |
| i.d. | R.S. mg/kg/h | 60,0 | 94,7 | | | 55,5 | | |
| 1.620 | Flow (ml/kg/h) | 2,53 ± 0,09 | 6,61 ± 0,27 | 5,94 ± 0,23 | 4,12 ± 0,14 | 3,23 ± 0,07 | 2,74 ± 0,06 | 2,23 ± 0,06 |
| 100 mg/kg | R.S. mg/ml | 30,2 | 25,7 | | | 23,3 | | |
| i.d. | R.S. mg/kg/h | 76,6 | 170,0 | | | 85,3 | | |
| Dehydrocholic acid | Flow (ml/kg/h) | 2,31 ± 0,15 | 3,82 ± 0,26 | 2,51 ± 0,17 | 2,63 ± 0,19 | 1,93 ± 0,12 | 1,87 ± 0,13 | 1,45 ± 0,14 |
| 50 mg/kg | R.S. mg/ml | 26,8 | 25,8 | | | 21,5 | | |
| i.d. | R.S. mg/kg/h | 61,9 | 98,5 | | | 44,5 | | |
| Dehydrocholic acid | Flow (ml/kg/h) | 1,80 ± 0,09 | 4,59 ± 0,24 | 2,45 ± 0,10 | 1,98 ± 0,09 | 1,82 ± 0,09 | 1,82 ± 0,08 | 1,55 ± 0,08 |
| 100 mg/kg | R.S. mg/ml | 29,0 | 32,0 | | | 25,9 | | |
| i.d. | R.S. | 52,3 | 147,0 | | | 49,9 | | |
| Dehydrocholic acid | Flow (ml/kg/h) | 2,28 ± 0,09 | 8,10 ± 0,38 | 3,76 ± 0,10 | 2,69 ± 0,07 | 2,77 ± 0,13 | 2,34 ± 0,11 | 2,16 ± 0,08 |
| 200 mg/kg | R.S. mg/ml | 31,1 | 31,4 | | | 24,4 | | |
| i.d. | R.S. mg/kg/h | 71,0 | 255,0 | | | 67,0 | | |

The same results have been expressed by means of graphs in order to make the conclusions which can be deduced from the tests more evident. In compiling the graphs, the differences between the choleresis in the treated animals and that in the controls was calculated over the considered time intervals, and expressed as a percentage of the base choleresis. For this calculation, the following formula was used:

$$\frac{C_n—C_b}{C_b} - \frac{C'_n—C'_b}{C'_b}$$

where

$C_n$ and $C'_n$ represent the choleresis (in ml/kg/h for the bile flow, and in mg/kg/h for the dry residue) determined over the considered time interval for the group of treated animals and the group of controls respectively. $C_b$ and $C'_b$ represent the base choleresis in the group of treated animals and group of controls respectively.

The graphs of Figure 1 show the increase in bile excretion over the controls, for rats treated with compound 1.620 in various orally administered doses.

In the graph A, the abscissa indicated the time in hours from the administration of the drug. The ordinate indicates the percentage increase in the bile flow. The curves I, II, III, IV relate respectively to doses of 25, 50, 100 and 200 mg/kg of compound 1.620.

In graph B, the white columns indicate the percentage increase in the bile flow at said increasing doses of compound 1.620, in the first hour after treatment, and the hatched columns indicate the increase in the dry residue in the same period and at the same doses.

The graph C indicates in the same way the percentage increase over the entire period of six hours following treatment.

The graphs A, B, C of Figure 2 give the same evaluation as graphs A, B, C of Figure 1, but for intraduodenal administration of 25 mg/kg (I), 50 mg/kg (II) and 100 mg/kg (III) of compound 1.620.

In Figure 3, graphs A, B, C, give the same evaluation as previously described, but for the oral administration of 25 mg/kg (I), 100 mg/kg (II), 200 mg/kg (III) and 400 mg/kg (IV) of dehydrocholic acid.

Finally, graphs A, B, C of figure 4 give the same evaluations for the oral administration of 200 mg/kg of dihydroxybutylether (I) and of 200 mg/kg of α-(1-hydroxy-4-phenylcyclohexyl)butyric acid (II).

The duration index has also been calculated for the choleretic product 1.620 in comparison with dehydrocholic acid, α-(1-hydroxy-4-phenylcyclohexyl)butyric acid and dihydroxybutylether.

This index was calculated as the time for halving the maximum choleresis increase recorded under their effect.

The results are shown in Table 4.

TABLE 4

| Treatment | Peak choleresis | | Duration index |
|---|---|---|---|
| | time of appearance | % increase | |
| 1620 (200 mg/kg p.o.) | 2nd hour | 135 | 240′ |
| Dehydrocholic acid | 1st hour | 94 | 45′ |
| α-(1-hydroxy-4-phenylcyclo-hexyl)-butyric acid | 1st hour | 105 | 180′ |
| Dihyroxydibutylether | 1st hour | 49 | 45′ |

In a second series of experiments on rats, the superimposability of the responses of compound 1.620 administered alone, as in the previous tests, and of the same compound administered in the complete speciality formulation (as CO) was tested, and in addition a comparison was made with scaler doses of dehydrocholic acid and α-(1-hydroxy-4-phenylcyclohexyl)butyric acid.

The results are shown in Table 5 and on the graphs of Figure 5.

On these graphs:

I = 25 mg of 1.620 administered gastrically as CO;
II = 25 mg of pure 1.620 administered gastrically;
III = 50 mg of 1.620 administered gastrically as CO;
IV = 50 mg of pure 1.620 administered gastrically;
V = 100 mg of 1.620 administered gastrically as CO;
VI = 100 mg of pure 1.620 administered gastically.

The abscissa axis represents time in hours from administration of the drug, and the ordinate axis represents the cumulative flow expressed as a percentage of the base flow.

TABLE 5

Action of CO, of compound 1.620, of sodium dehydrocholate (A) and $\alpha$-(1-hydroxy-4-phenyl-cyclohexyl)-butyric acid (V) on choleresis of the rat.

| An. n | Oral treatment (by gastric probe-) mg/kg | ml of bile flow ± s.e. at the following times from the administration of the drug 0(base values) | 1h | 2h | 3h | 4h | 5h | 6h |
|---|---|---|---|---|---|---|---|---|
| 10 | Controls | 2,6 ± 0,15 | 2,6 ± 0,18 | 1,9 ± 0,29 | 1,8 ± 0,16 | 1,7 ± 0,15 | 1,6 ± 0,10 | 1,5 ± 0,11 |
| 10 | CO (25) | 2,5 ± 0,16 | 3,2 ± 0,20 | 2,9 ± 0,21 | 2,5 ± 0,22 | 2,0 ± 0,19 | 1,9 ± 0,11 | 1,8 ± 0,10 |
| 10 | CO (50) | 2,4 ± 0,20 | 3,4 ± 0,40 | 3,1 ± 0,19 | 2,9 ± 0,20 | 2,8 ± 0,18 | 2,7 ± 0,15 | 2,4 ± 0,14 |
| 10 | CO (100) | 2,7 ± 0,19 | 5,1 ± 0,49 | 4,0 ± 0,38 | 3,6 ± 0,52 | 3,2 ± 0,41 | 2,9 ± 0,20 | 2,8 ± 0,20 |
| 10 | 1,620 (25) | 2,6 ± 0,20 | 3,2 ± 0,14 | 2,7 ± 0,10 | 2,6 ± 0,21 | 1,9 ± 0,11 | 1,6 ± 0,20 | 1,6 ± 0,21 |
| 10 | 1.620 (50) | 2,8 ± 0,19 | 3,7 ± 0,31 | 3,4 ± 0,22 | 3,2 ± 0,19 | 3,0 ± 0,18 | 2,8 ± 0,14 | 2,2 ± 0,18 |
| 10 | 1,620 (100) | 2,5 ± 0,16 | 4,8 ± 0,26 | 3,7 ± 0,31 | 3,5 ± 0,20 | 3,2 ± 0,32 | 2,9 ± 0,22 | 2,7 ± 0,23 |
| 10 | A (25) | 2,4 ± 0,21 | 3,0 ± 0,25 | 2,0 ± 0,12 | 1,9 ± 0,11 | 1,7 ± 0,13 | 1,7 ± 0,10 | 1,4 ± 0,11 |
| 10 | A (50) | 2,5 ± 0,16 | 3,9 ± 0,18 | 2,1 ± 0,16 | 1,9 ± 0,14 | 1,8 ± 0,17 | 1,7 ± 0,11 | 1,5 ± 0,13 |
| 10 | V (50) | 2,8 ± 0,22 | 3,2 ± 0,11 | 3,0 ± 0,12 | 2,9 ± 0,10 | 2,8 ± 0,12 | 2,5 ± 0,10 | 2,0 ± 0,11 |
| 10 | V (100) | 2,6 ± 0,17 | 3,7 ± 0,13 | 3,6 ± 0,14 | 3,4 ± 0,11 | 3,0 ± 0,11 | 2,9 ± 0,13 | 2,1 ± 0,13 |

Finally, in a third series of tests on rats, the increase in the bile flow, dry residue and cholesterol excreted were evaluated after administering 50 mg/kg of 1.620 as CO.

The increases were calculated relative to the controls and expressed as a percentage of the base values.

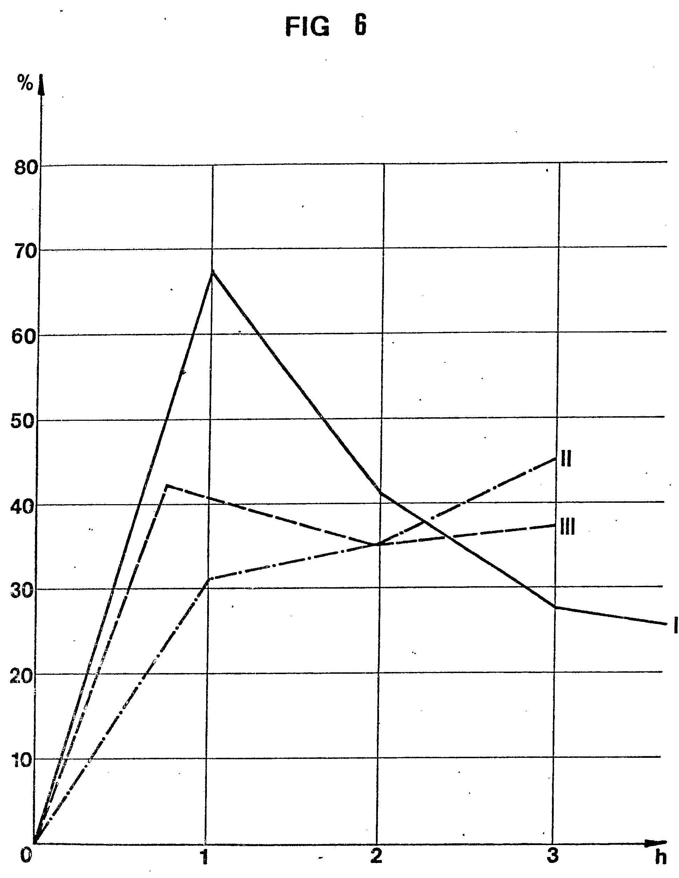

In Figure 6, curve I relates to the bile flow, curve II to the dry residue and curve III to the total cholesterol.

The $DL_{50}$ for the $\alpha$-methyl-$\alpha$-(3,4-dihydro-1-naphtyl)acetic acid compound (1.620) determined in the musmusculus had the following values:

$DL_{50}$ by oral administration = 1663 (1451—1905) mg/kg
$DL_{50}$ by i.p. administration = 1383 (1226—1559) mg/kg

The determination in the rat gave the following values:

$DL_{50}$ by oral administration = 1720 (1520—1921) mg/kg
$DL_{50}$ by i.p. administration = 1339 (1270—1744) mg/kg

Large doses of $\alpha$-methyl-$\alpha$-(3,4-dihydro-1-naphthyl)acetic acid (compound 1.620) which were many times greater than those obtainable in therapy, i.e. doses of 50 mg/kg/die administered for 180 consecutive days to the rat, were well tolerated and were free from effects on the main organs, systems and functions.

The organ tolerability with regard to normal livers, livers subjected to the action of inducing and hepatotoxic drugs, and livers strongly traumatised as a result of partial hepatoectomia was excellent.

All the aforesaid results were confirmed in the dog (and agree with the clinical results obtained).

The single human therapeutic dose per kg of body weight has been determined as 1.66 mg of compound 1.620, and the human therapeutic dose per kg of body weight per day has been determined as 4.99 mg of compound 1.620.

The following conclusion can be drawn from the total research carried out on the choleretic product 1.620, and of which only some significant data have been given heretofore.

1) In the most common laboratory animals (rat, mus musculus and dog), the product 1.620 has a very low acute, sub-acute and chronic toxicity, because of which it is well tolerated orally (its normal method of human clinical administration), even at large doses which are many times greater than those recommended or attainable in clinical practice.

2) The local tolerability of the product by the gastroenteric mucosa appeared satisfactory in the albina rat and dog, even for repeated administrations over 180 consecutive days at doses which per kg and per day were many times greater than the human therapeutical doses.

3) The product 1.620 proved to be well tolerated by a normal liver, by a liver subjected to inducing drugs (phenobarbitone) or hepatoxic drugs (carbon tetrachloride, allyl alcohol, $\alpha$-naphthyl-isothiocyanate), and by a liver strongly traumatised by partial hepatectomia, even at doses which are considerably greater than the therapeutic dose. In addition, it gives rise to no significant pressor, respiratory or ecgraphic modifications, or modifications of the VNS relative to the cardiovascular system, nor does it have any significant depressive or stimulating effects on the CNS.

4) The product 1.620 has choleretic effects characterises by considerable intensity and in particular by long duration, these being considerably greater than those observed for dehydrocholate and other synthetic choleretics of current therapeutic use.

5) In addition to its choleretic effect, the product 1.620 has moderate laxative effects and an undoubted cholesterol-lowering and antidislipidemic action. According to the results of certain tests, it also seems able to reduce the accumulation of cholesterol and hepatic triglycerides.

The elevated biligenetic activity of the product 1.620 enables the desired pharmacological effect to be obtained by smaller dosages than those commonly used in the case of other choleretics, and which are far removed from doses which could cause toxic effects, by virtue of the low toxicity of the drug.

The elevated biligenetic activity of the drug derives not only from the intensity of its action, which is greater than the greater duration of the biligenetic affect in particular.

This is particularly useful at the applicational level, in that it enables an effective increase in the bile fluid to be obtained which is better distributed over the entire period of time during which it is desirable that it should occur, i.e. during the two to three hours corresponding to the average gastric emptying time after a regular meal.

In addition to the choleretic effect, the mild laxative effect is advantageous for therapeutic reasons, as it is capable of reducing the stypsis which normally occurs during bile insufficiency, and the protective effect against hepatic steatosis is also advantageous, and on which the deficit in the biligenetic function can at least partly depend.

The perfect tolerability by the organ concerned in the biligenetic function, in addition to the low acute and chronic toxicity, constitute further advantages in the clinical use of the choleretic product 1.620.

α-methyl-α-(3,4-dihydro-1-naphthyl)acetic acid, the new and unforeseeable therapeutic use of which is the subject matter of the present invention, has been prepared starting from tetralone and a α-bromopropionate by way of the following reaction scheme:

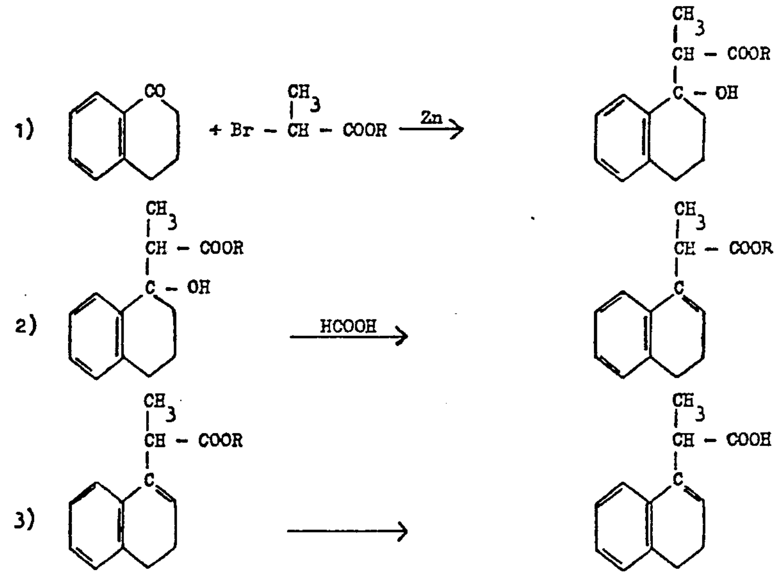


in which R is a linear or branched alkyl radical of 1 to 4 carbon atoms.

The first stage is carried out by heating to boiling point a solution of the two reagents in an inert organic solvent, in the presence of zinc filings. The reagents are mixed in proportions which are preferably stoichiometric, but at least close to stoichiometric.

A few iodine crystals can be added to the solution in order to accelerate the initiation of the reaction.

The solution is kept boiling for approximately a further hour after the end of the exothermic stage.

After the solution has been cooled, it is washed first with dilute $H_2SO_4$, then with a solution of sodium bicarbonate and finally with water.

The ester of α-methyl-α-(1-hydroxy-1,2,3,4-tetrahydro-1-naphthyl)-acetic acid is then separated by distillation.

To carry out the second stage of the process, the ester thus separated is dissolved in 99% formic acid and is kept for 30—60 minutes over a boiling water bath.

After the solution has been cooled, it is repeatedly extracted with benzene (or an equivalent organic solvent), and the benzene extracts are distilled under vacuum to give the ester of α-methyl-α-(3,4-dihydro-1-naphthyl)-acetic acid.

Finally, α-methyl-α-(3,4-dihydro-1-naphthyl)-acetic acid is released from the ester by alkaline hydrolysis, preferably with alcoholic soda or potash, under reflux.

The free acid is separated from the cooled and acidified solution by crystallisation.

One working example is described hereinafter in order to provide all the operational details and facilitate reproduction of the process according to the present invention.

Example

1) Ethyl ester of α-methyl-α-(1-hydroxy-1,2,3,4-tetrahydro-1-naphthyl)-acetic acid from α-tetraline and ethyl α-bromopropionate

15.7 g (0.24 moles) of zinc filings are suspended in 170 ml of anhydrous benzene. 38 g (0.21 moles) of ethyl α-bromopropionate are added, and the mixture is heated under agitation until boiling begins. 29.2 g (0.2 moles) of α-tetralone are then dripped in, while controlling the addition and heating in such a manner as to maintain the mixture under strong but not fierce boiling. The addition of a few iodine crystals can accelerate the reaction, should it proceed too slowly. When the exothermic stage of the reaction ceases, boiling under agitation for one hour. The mixture is cooled, the benzene solution is decanted to leave behind the unreacted zinc, and is shaken with about 800 ml of 0.5 N $H_2SO_4$ cooled to 5°C.

The extraction is repeated with a further portion of dilute $H_2SO_4$ if an aqueous extract does not give a clearly acid reaction. The benzene phase is then washed by shaking with 100 ml of a saturated sodium bicarbonate solution, and then with water. It is then dehydrated with sodium sulphate and is then distilled under vacuum, with firstly the benzene distilling off then the residue, and that fraction of the residue which passes over in the range 130—140°C at 3,75—$10^4$ Pa (0.5 mm Hg) is collected. Approximately 35 g of crude ethyl ester of α-methyl-α-(1-hydroxy-1,2,3,4-tetrahydro-1-naphthyl)-acetic acid are obtained, and after further distillation under vacuum, appear as a colourless liquid having a boiling point of 136°C at 3,75.10[4] Pa (0.5 mm Hg).

C% calculated 72.55 Found 72.40
H% calculated 8.12 Found 8.26

2) Ethyl ester of $\alpha$-methyl-$\alpha$-(3,4-dihydro-1-naphthyl)-acetic acid from the ethyl ester of $\alpha$-methyl-$\alpha$-(1-hydroxy-1,2,3,4-tetrahydro-1-naphthyl)-acetic acid

24.8 g (0.1 moles) of the ethyl ester of $\alpha$-methyl-$\alpha$-(1-hydroxy-1,2,3,4-tetrahydro-1-naphthyl)-acetic acid are dissolved in 75 ml of 99% formic acid and kept over a boiling water bath for 30 to 60 minutes. It is cooled, poured into water cooled to 5°C, and then extracted firstly with 100 ml and then with 50 ml of benzene. The benzene extracts are collected and shaken with 50 ml of a saturated solution of sodium bicarbonate, then with 50 ml of water. The benzene solution is dehydrated with sodium sulphate and is then distilled under vacuum, with firstly the benzene and then the residue distilling over, and that fraction of this latter which passes over in the range of 115—125°C at 2,25.10[4] Pa (0.3 mm Hg) is collected.

About 20 g of the crude ethyl ester of $\alpha$-methyl-$\alpha$-(3,4-dihydro-1-naphtyl)-acetic acid is collected, and when purified by further distillation under vacuum appears as a slightly yellow coloured liquid having a B.P. of 119—120°C at 2,25.10[4] Pa (0.3 mm Hg).

C% calculated 78.22 found 77.98
H% calculated 7.88 found 7.90

3) $\alpha$-methyl-$\alpha$-(3,4-dihydro-1-naphthyl)-acetic acid from the ethyl ester of $\alpha$-methyl-$\alpha$-(3,4-dihydro-1-naphthyl)-acetic acid

23 g (0.1 moles) of the ethyl ester of $\alpha$-methyl-$\alpha$-(3,4-dihydro-1-naphthyl)-acetic acid are dissolved in 213 ml of 50% aqueous ethanol containing 6.8 g (0.17 moles) of NaOH.

The solution is kept under reflux for 4 hours, is then cooled, brought to pH 8 with concentrated HCl, and the alcohol is then evaporated under vacuum.

The residual alkaline solution is diluted with an equal volume of water, treated with 2% of decolourising carbon, filtered and acidified carefully under cooling with the necessary quantity of concentrated HCl.

An oil separates which rapidly transforms into a solid mass, which is then collected at the pump and crystallised firstly from 70% ethyl alcohol and then from ligroin.

Approximately 14 g of $\alpha$-methyl-$\alpha$-(3,4-dihydro-1-naphthyl)-acetic acid are obtained in the form of white crystals having a M.P. of 114—116°C.

C% calculated 77.20 found 77.13
H% calculated 6.98 found 7.18

**Claim**

Therapeutic composition for use as a choleretic medicament, which contains as its active compound $\alpha$-methyl-$\alpha$-(3,4-dihydro-1-naphthyl)-acetic acid of formula

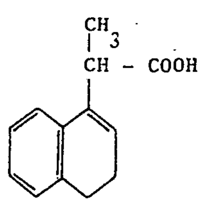

or a salt thereof.

**Patentanspruch**

Therapeutische Mittel zur Verwendung als choleretisches Medikament, welche also aktiven Verbindung $\alpha$-Methyl-$\alpha$-(3,4-dihydro-1-naphtyl)-essigsäure der Formel

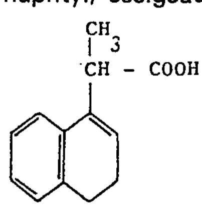

oder eines ihrer Sakze enthalten.

**Revendication**

Composition thérapeutique destinée à être utilisée comme médicament cholérétique, caracterisée en ce qu'elle contient, en tant que composé actif, un acide -méthyl-(3,4-dihydro-1-naphtyl)-acétique de formule

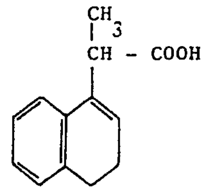

ou un sel de cet acide.

FIG 1

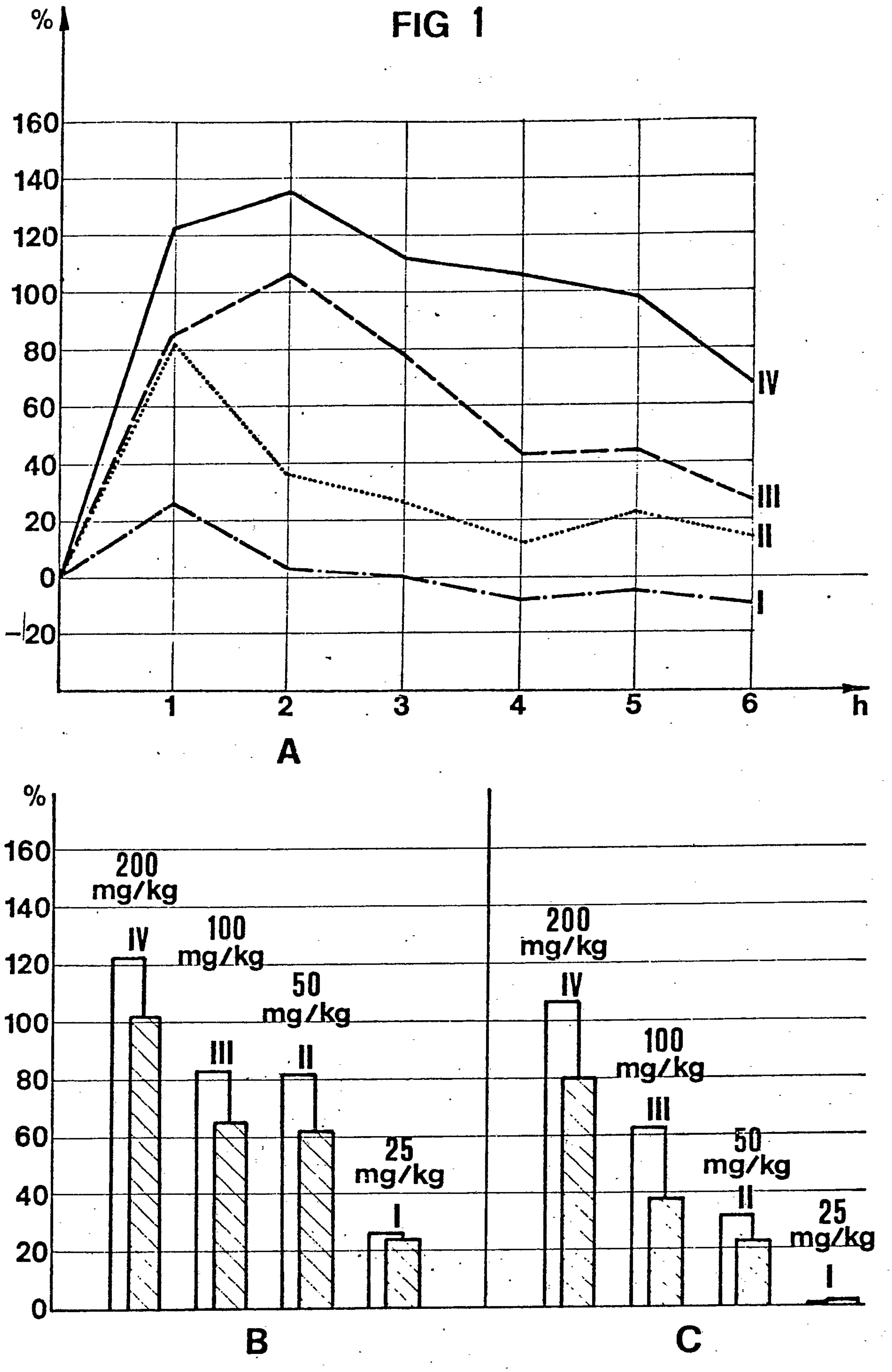
%
160
140
120
100
80
60
40
20
0
-20
IV
III
II
I
1
2
3
4
5
6
h
A
%
160
140
120
100
80
60
40
20
0
200 mg/kg
IV
100 mg/kg
III
50 mg/kg
II
25 mg/kg
I
200 mg/kg
IV
100 mg/kg
III
50 mg/kg
II
25 mg/kg
I
B
C

FIG 2

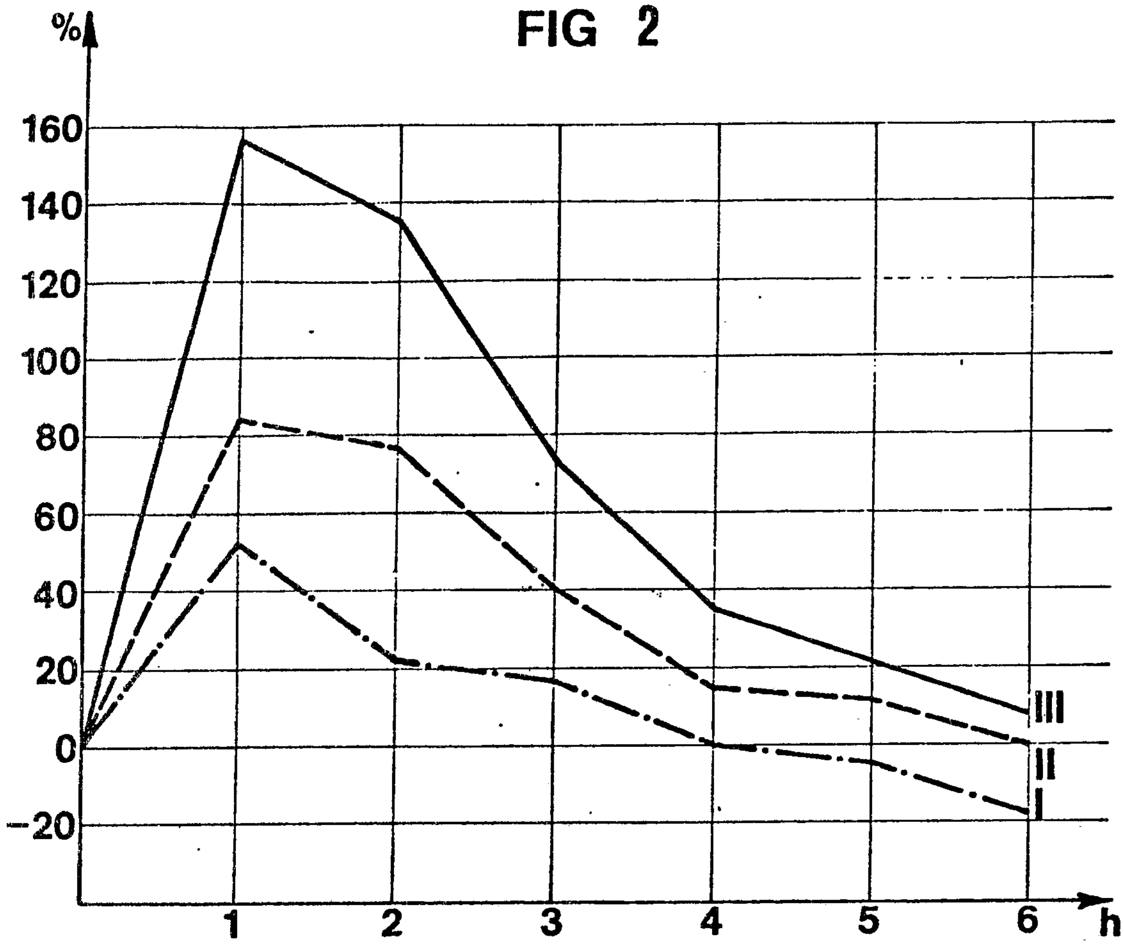
%
160
140
120
100
80
60
40
20
0
−20
1
2
3
4
5
6
h
III
II
I

A

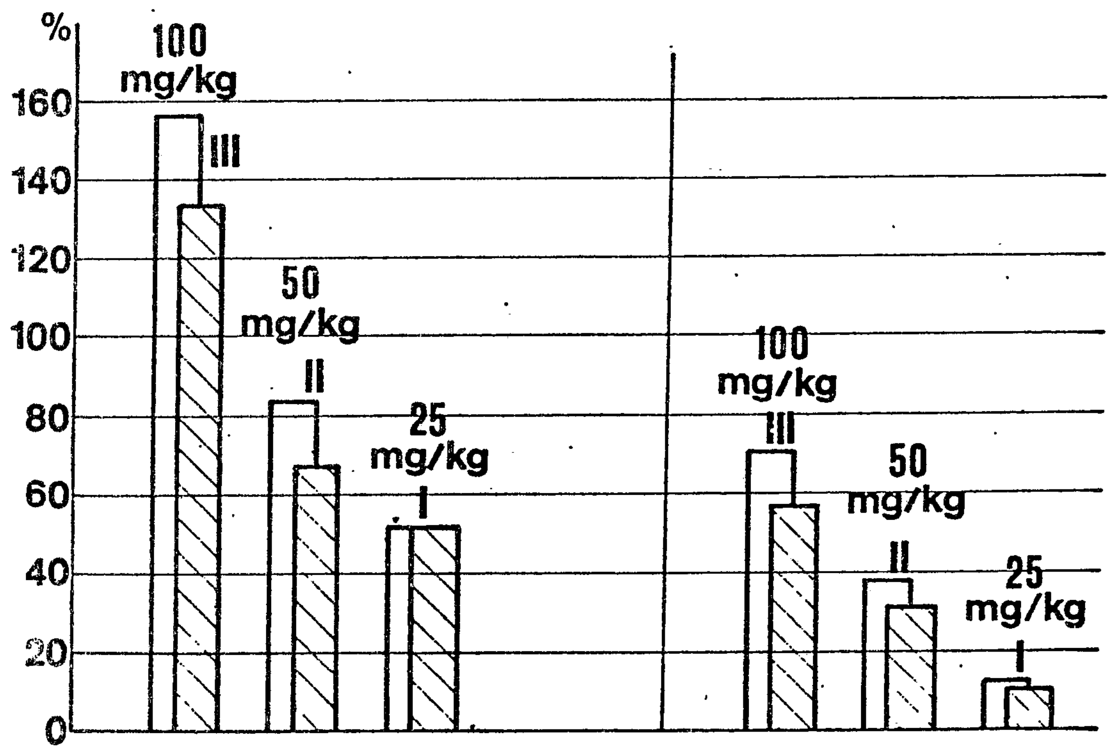
%
160
140
120
100
80
60
40
20
0
100 mg/kg
III
50 mg/kg
II
25 mg/kg
I
100 mg/kg
III
50 mg/kg
II
25 mg/kg
I

B

C

FIG 3

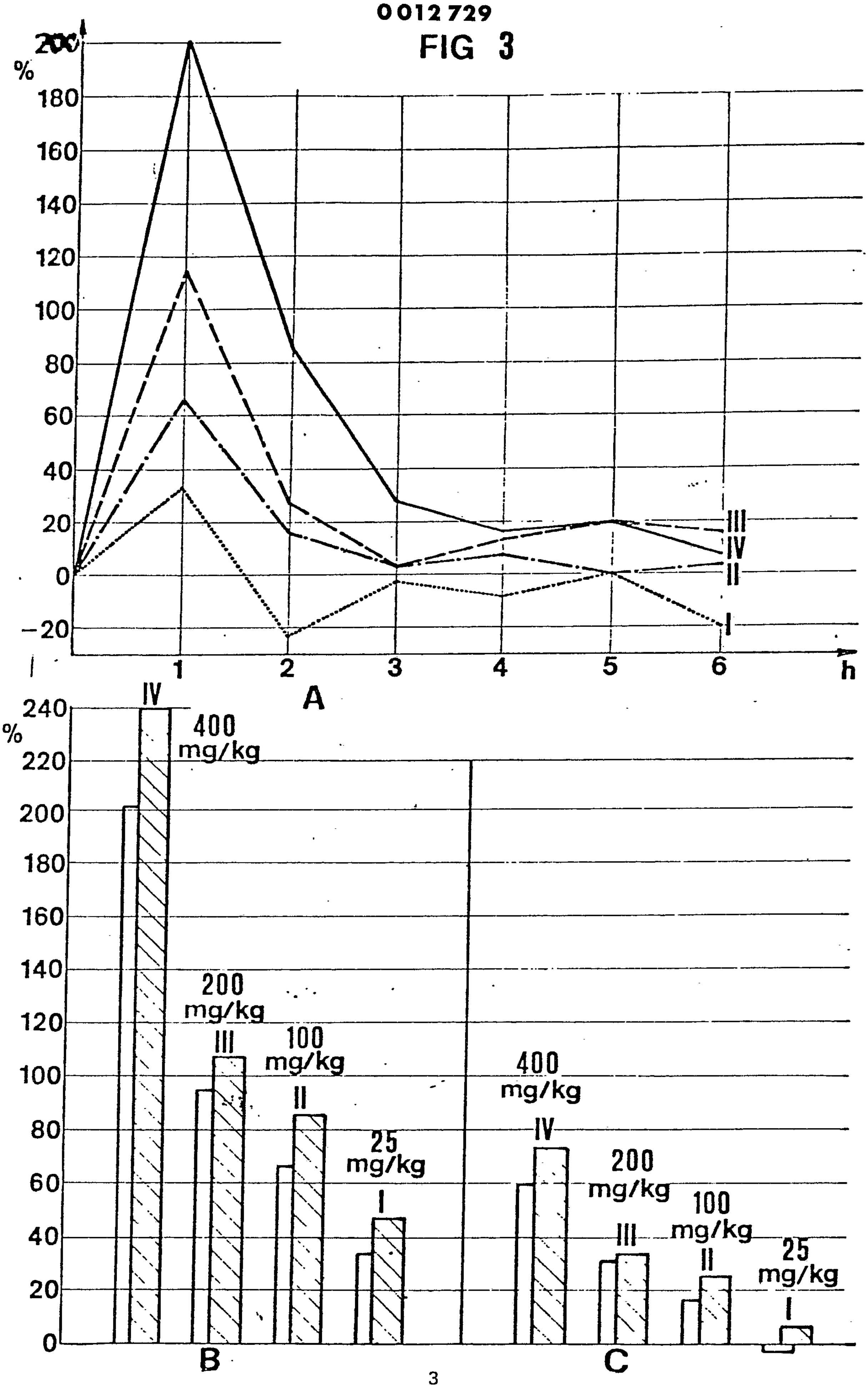
%
200
180
160
140
120
100
80
60
40
20
0
−20
1
2
3
4
5
6
h
III
IV
II
I
A
%
240
220
200
180
160
140
120
100
80
60
40
20
0
IV
400 mg/kg
200 mg/kg
III
100 mg/kg
II
25 mg/kg
I
400 mg/kg
IV
200 mg/kg
III
100 mg/kg
II
25 mg/kg
I
B
C

FIG 4

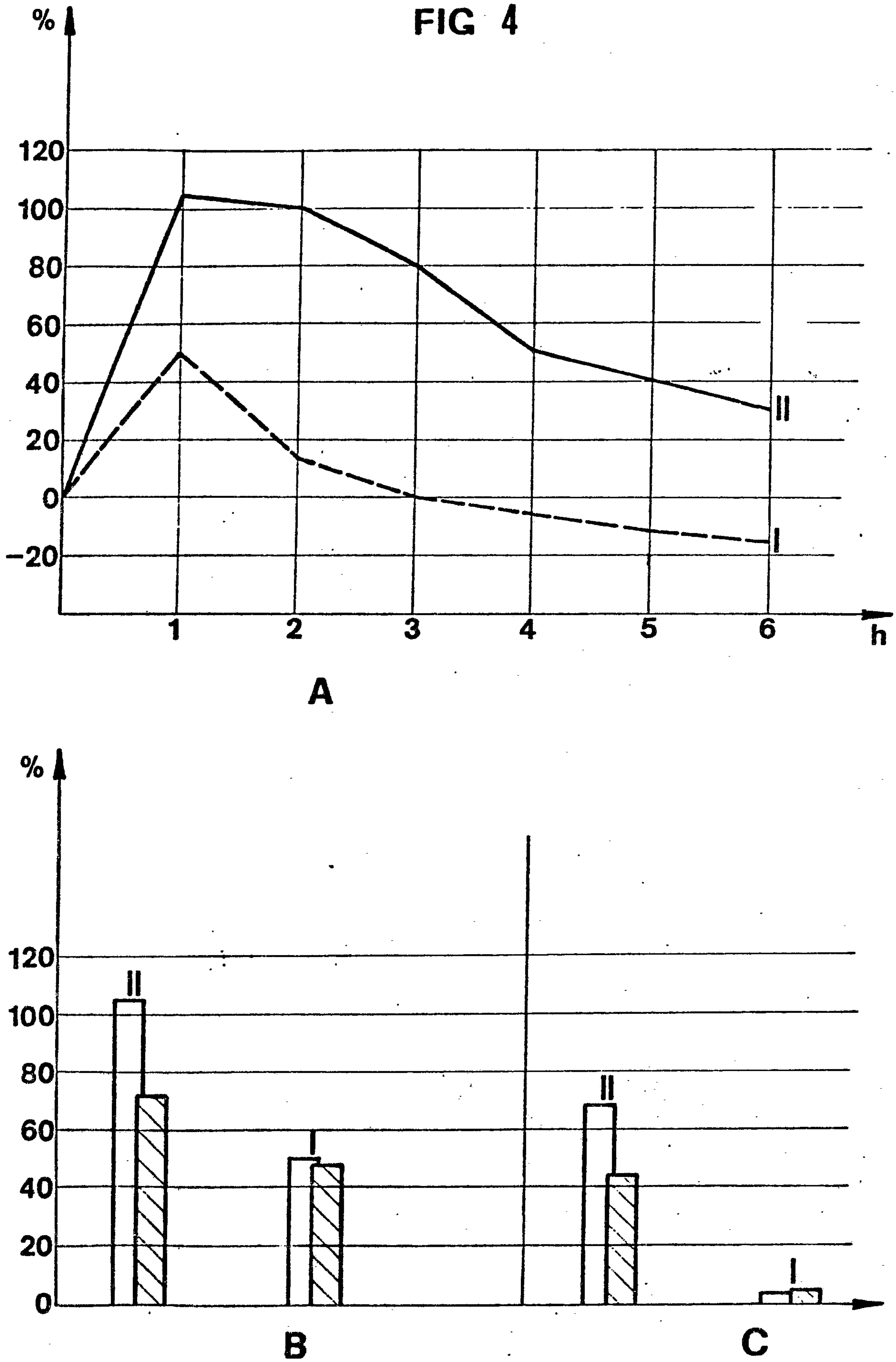
%
120
100
80
60
40
20
0
−20
1
2
3
4
5
6
h
II
I
A
%
120
100
80
60
40
20
0
II
I
II
I
B
C

FIG 5

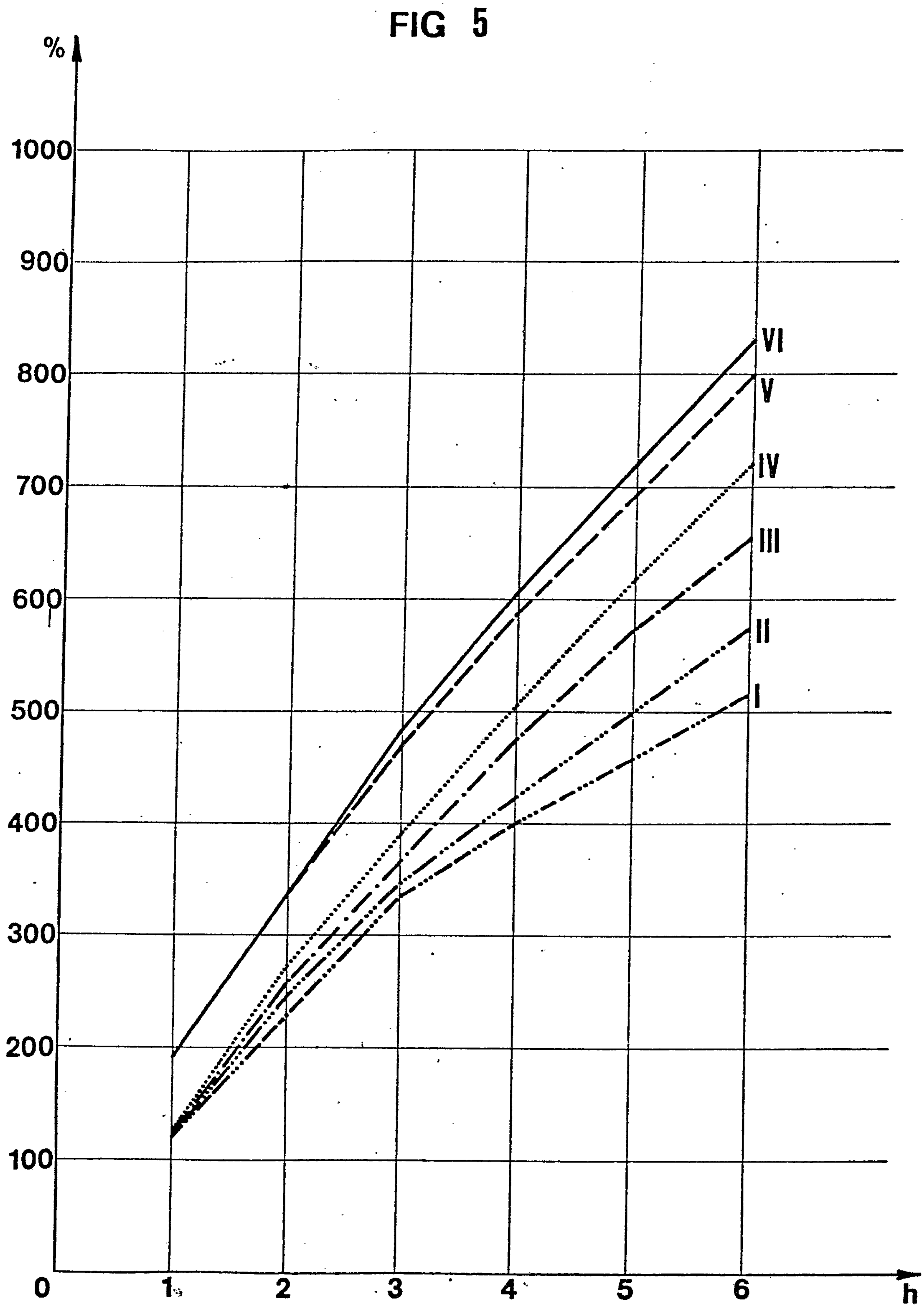
%
1000
900
800
700
600
500
400
300
200
100
0
1
2
3
4
5
6
h
VI
V
IV
III
II
I

FIG 6